# EUROPÄISCHE PATENTSCHRIFT

(11) **EP 3 077 493 B1**
(45) Veröffentlichungstag und Bekanntmachung des Hinweises auf die Patenterteilung: **29.08.2018**
(21) Anmeldenummer: 14814762.2
(22) Anmeldetag: 03.12.2014
(51) Int. Cl.: C11D 3/34, C11D 10/04, C11D 17/00, C11D 1/655

(54) **GLUCAMIDE IN SYNDETSEIFEN**
GLUCAMIDES IN SYNDETS
GLUCAMIDE DANS DES SAVONS SYNDETS

(30) Priorität: 03.12.2013 US 201361911003 P
(43) Veröffentlichungstag der Anmeldung: 12.10.2016
(73) Patentinhaber: Clariant International Ltd, 4132 Muttenz (CH)
(72) Erfinder: KLUG, Peter, 63762 Grossostheim (DE); DAHMS, Gerd, 47138 Duisburg (DE); GANDOLFI, Lisa Renne, Charlotte, NC 28273 (US)
(74) Vertreter: Holmes, Rosalind
(86) Internationale Anmeldenummer: PCT/EP2014/003215
(87) Internationale Veröffentlichungsnummer: WO 2015/082062

(56) Entgegenhaltungen:
- WO-A1-98/06800
- DE-C1- 19 645 214

## Beschreibung

Die Erfindung betrifft Seifenstücke enthaltend N-Alkyl-N-Acylglucamine, Fettsäuren und/oder Seifen, Acylisethionate und Natriumisethionat. Ferner betrifft die Erfindung deren Verwendung als Combibar oder Syndetbar, die Verwendung zur Behandlung oder Pflege der Haut oder Haare, sowie ein Verfahren zur Herstellung eines erfindungsgemäßen Seifenstücks Seifenstücke spielen bei der Körperreinigung und Pflege seit jeher eine große Rolle. Klassische Seifen stellen dabei die sogenannten Alkaliseifen dar. Alkaliseifen enthalten ausschließlich Fettsäuresalze und gegebenenfalls noch freie Fettsäuren als Verunreinigung. Die Fettsäuresalze bilden sich durch Umsetzung von Fettsäuren mit einer Lauge, z.B. Kalilauge oder Natronlauge, in einer Verseifungsreaktion.
Neben den klassischen Alkaliseifen, werden heutzutage auch sogenannte Combibars angeboten. Combibars sind Seifenstücke, die neben Fettsäuresalzen noch weitere synthetische Tenside (i.d.R. Fettalkoholethersulfate oder Fettsäureisethionate) aufweisen.
Zunehmend an Bedeutung gewinnen die sogenannten Syndetbars. Syndetbars sind Seifenstücke, die frei von Fettsäuresalzen sind und ausschließlich synthetische Tenside aufweisen. Ihnen wird eine besonders gute Hautverträglichkeit und ein geringes allergieauslösendes Potential zugeschrieben.

Im Allgemeinen werden an Seifenstücke heutzutage hohe Anforderungen gestellt. Die Seifenstücke sollen nicht nur eine reinigende Wirkung, sondern auch pflegende Eigenschaften aufweisen. Ein Seifenstück soll also eine angenehme Haptik aufweisen und besonders viel cremigen Schaum bei der Anwendung erzeugen.
Die Aufgabe der Erfindung liegt somit darin, verbesserte Zusammensetzungen bereitzustellen, die bei Ihrer Verwendung als Seifenstück eine erhöhte Schaummenge produzieren, eine größere Härte aufweisen und zu einer Verringerung der Rauigkeit führen.
Die Verwendung von N-Alkyl-N-Acylglucaminen und Isethionaten bei der Seifenherstellung ist schon lange bekannt.
Die WO 98/05752 und die WO 92/13059 offenbaren Seifenstücke, die Alkyl-N-Methylglucamide aufweisen.
In der WO 98/15606 werden fettsäurefreie Syndetseifen beschrieben, die Fettsäure-N-alkylglucamide und Fettsäureisethionate enthalten.
Die WO 01/72946, die DE 19645214 und die WO 01/72947 beschreiben Seifen aus Fettsäure-N-Alkylpoly-hydroxyalkylamiden und Kokosfettsäureisethionat Na-Salz. Die Kombination mit Kokosfettsäureisethionat NH₄-Salz ist in der WO 95/07975 offenbart.
Die WO 98/00492 als auch die WO 98/06800 sind auf Seifenstücke gerichtet, die Glucamide und Acylisethionate enthalten.

Auch die DE 19703745 offenbart Stückseifen. Die hier beschriebenen Zusammensetzungen enthalten Fettsäure-N-Alkylglucamide und Fettsäuren.
Es wurde nun gefunden, dass Zusammensetzungen, die neben einem N-Alkyl-N-Acylglucamin, einer Fettsäure und/oder Seife und einem Acylisethionat Natriumisethionat enthalten, sich für den Einsatz als Stückseifen eignen. Eine solche Eignung lässt sich aus keinem der genannten Dokumente herleiten.

Demgemäß wird ein Seifenstück bereitgestellt, enthaltend:
- mindestens ein N-Alkyl-N-Acylglucamin als Komponente A,
- mindestens eine Fettsäure und/oder Seife als Komponente B,
- mindestens ein Acylisethionat als Komponente C,
- Natriumisethionat als Komponente D,
- Wasser als Komponente E,
- optional mindestens ein weiteres Additiv als Komponente F,
wobei mehr als 20 Gew.%, bevorzugt mehr als 70 Gew.% der N-Alkyl-N-Acylglucamine mindestens eine C₁₂- und/oder C₁₄- und/oder C₁₆- und/oder C₁₈-Acylgruppe enthalten.

In einer bevorzugten Ausführungsform enthält in einem erfindungsgemäßen Seifenstück die Komponente A N-Alkyl-N-Acylglucamine, wobei mehr als 20 Gew.%, bevorzugt mehr als 70 Gew.% der N-Alkyl-N-Acylglucamine mindestens eine C₁₂- und/oder C₁₄-Acylgruppe enthalten.

Die erfindungsgemäße Zusammensetzung des Seifenstücks weist vorteilhafterweise eine erhöhte Schaumbildung, eine erhöhte Härte und eine verringerte Rauigkeit auf.

Erfindungsgemäß bevorzugt ist eine Zusammensetzung desSeifenstücks enthaltend:
- 0,5-5,0 Gew.% der Zusammensetzung an Komponente A,
- 25,0-50,0 Gew.% der Zusammensetzung an Komponente B,
- 28,0-50,0 Gew.% der Zusammensetzung an Komponente C,
- 2,0-10,0 Gew.% der Zusammensetzung an Komponente D.

Besonders bevorzugt ist eine Zusammensetzung des Seifenstücks enthaltend:
- 1,0-3,0 Gew.% der Zusammensetzung an Komponente A,
- 25,0-35,0 Gew.% der Zusammensetzung an Komponente B,
- 40,0-48,0 Gew.% der Zusammensetzung an Komponente C,
- 3,0-6,0 Gew.% der Zusammensetzung an Komponente D.

Die erfindungsgemäß verwendeten N-Alkyl-N-Acylglucamine, bei denen Glucamin vorzugsweise eine N-1-Desoxysorbitylgruppe bedeutet, sind besonders bevorzugt N-Alkyl-N-Acylglucamine der Formel (I), 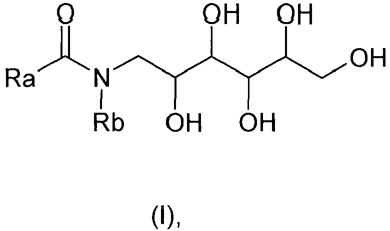 wobei in der Formel (I) RₐCO einen linearen oder verzweigten, gesättigten oder ungesättigten C₈-C₂₂-Acylrest und R_{b} einen C₁-C₄ Alkylrest bedeutet. Besonders bevorzugt bedeutet R_{b} in Formel (I) einen Methylrest (-CH₃) und RₐCO hat vorstehende Bedeutung.

Bevorzugt als N-Alkyl-N-Acylglucamine sind Verbindungen der Formel (I), bei denen RₐCO einen C₁₂-C₁₈-Acylrest bedeutet. Insbesondere bevorzugt sind N-Alkyl-N-Acylglucamine der Formel (I), bei denen RₐCO einen C₁₂-C₁₈-Acylrest bedeutet und R_{b} einen Methylrest bedeutet.

Besonders bevorzugt liegt der Anteil an N-Alkyl-N-Acylglucaminen, die eine C₁₂- und/oder C₁₄- und/oder C₁₆- und/oder C₁₈- Acylgruppe, insbesondere eine C₁₂- und/oder C₁₄-Acylgruppe enthalten, bei mindestens 70 Gew.-% und der Anteil an N-Alkyl-N-Acylglucaminen, die eine Acylgruppe < C₁₂ enthalten bei weniger als 3 Gew.-%.

Insbesondere bevorzugt liegt der Anteil an N-Alkyl-N-Acylglucaminen, die eine C₁₂- und/oder C₁₄- und/oder C₁₆- und/oder C₁₈- Acylgruppe, insbesondere eine C₁₂- und/oder C₁₄-Acylgruppe enthalten, bei mindestens 80 Gew.-%. Bevorzugt liegt gleichzeitig der Anteil an N-Alkyl-N-Acylglucaminen, die eine Acylgruppe < C₁₂ enthalten bei weniger als 2 Gew.-%.

In einer weiteren Ausführungsform liegt der Anteil an N-Alkyl-N-Acylglucaminen, die eine C₁₂- und/oder C₁₄- und/oder C₁₆- und/oder C₁₈- Acylgruppe, insbesondere eine C₁₂- und/oder C₁₄-Acylgruppe enthalten, bei mindestens 90 Gew.-%. Bevorzugt liegt gleichzeitig der Anteil an N-Alkyl-N-Acylglucaminen, die eine Acylgruppe < C₁₂ enthalten bei weniger als 2 Gew.-%.

Im Rahmen einer weiteren bevorzugten Ausführungsform besteht die Komponente A aus einer Mischung aus N-Alkyl-N-Acylglucaminen. Bevorzugt handelt es sich bei der Mischung um eine Mischung, die mindestens ein N-Alkyl-N-C₈-C₂₂-Acylglucamin enthält, besonders bevorzugt mindestens ein N-Methyl-N-C₈-C₂₂-Acylglucamin.

Bevorzugt als Komponente A sind gesättigte N-Alkyl-N-Acylglucamine der Formel (I), wobei der Acylrest RₐCO abgeleitet ist von Myristinsäure, Laurinsäure, Palmitinsäure, Stearinsäure, Ölsäure, Linolsäure oder Linolensäure.

Bevorzugt sind auch N-Alkyl-N-Acylglucamine der Formel (I), bei denen RₐCO abgleitet ist von Kokosöl.

Kokosöl enthält typischerweise Triglyceride, die gesättigte Fettsäurereste enthalten, die sich von Capryl-, Laurin-, Caprin-, Öl-, Palmitin-, Stearin- und Myristinsäure ableiten.

Kokosöl umfasst dabei vorzugsweise
a) 40-55 Gew.-% Laurinsäure,
b) 10-20 Gew.-% Myristinsäure,
c) 8-12 Gew.-% Palmitinsäure,
d) 6-12 Gew.-% Ölsäure und
h) 0-36 Gew.-% weiterer Fettsäuren,
wobei die Summe der an das Triglycerid gebundenen Fettsäuren 100 Gew.-% ergibt.

Besonders bevorzugt umfasst Kokosöl
a) 40-55 Gew.-% Laurinsäure,
b) 10-20 Gew.-% Myristinsäure,
c) 8-12 Gew.-% Palmitinsäure,
d) 6-12 Gew.-% Ölsäure,
e) 5-10 Gew.-% Decansäure,
f) 4-10 Gew.-% Octansäure,
g) 1-3 Gew.-% Stearinsäure und
h) 0-26 Gew.-% weiterer Fettsäuren,
wobei die Summe der an das Triglycerid gebundenen Fettsäuren 100 Gew.-% ergibt.

Neben den N-Alkyl-N-Acylglucaminen der Formel (I), bei denen RₐCO einen C₁₂-C₁₄-Acylrest bedeutet, können die Zusammensetzungen geringe Anteile an von kurzkettigen und/oder langkettigen Fettsäuren abgeleiteten N-Alkyl-N-Acylglucamine aufweisen, insbesondere solche, welche C₁-C₄-Acyl, C₆-, C₈-, C₁₀-, C₁₆-, C₁₈- und/oder C₂₀-Acyl enthalten.

Besonders bevorzugt ist das Gewichtsverhältnis von N-Alkyl-N-Acylglucamin der Formel (I), wobei RₐCO einen C₁₂-Alkylrest bedeutet zu N-Alkyl-N-Acylglucamin der Formel (I), wobei RₐCO einen C₁₄-Alkylrest bedeutet, 50 : 50 bis 90 : 10, insbesondere 60 : 40 bis 80 : 20.

In einer weiteren Ausführungsform ist das N-Alkyl-N-Acylglucamin einer erfindungsgemäßen Zusammensetzung eine Mischung aus mindestens einem N-Alkyl-N-Acylglucamin der Formel (I), wobei RₐCO einen C₁₆-Acylrest bedeutet und mindestens einem N-Alkyl-N-Acylglucamin der Formel (I), wobei RₐCO einen C₁₈-Acylrest bedeutet.

Bevorzugt beträgt der Anteil an Komponente A in einer erfindungsgemäßen Zusammensetzung 0,5-5,0 Gew.%, bezogen auf die Zusammensetzung, und besonders bevorzugt 1,0-3,0 Gew.%, bezogen auf die Zusammensetzung.

Die hier eingesetzten N-Alkyl-N-Acylglucamine können, wie in EP 0 550 637 A1 beschrieben, durch Umsetzung der entsprechenden Fettsäureester oder Fettsäureestergemische mit N-Alkylglucamin in Gegenwart eines Hydroxylgruppen oder Alkoxylgruppen aufweisenden Lösungsmittels hergestellt werden: Geeignete Lösungsmittel sind beispielsweise C₁-C₄-Monoalkohole, Ethylenglykol, Propylenglykol, Glycerin sowie alkoxylierte Alkohole. Bevorzugt ist 1,2-Propylenglykol. N-Alkylglucamin kann, wie ebenfalls in EP 0 550 637 A1 beschrieben, durch reduktive Aminierung von Glukose mit Alkylamin erhalten werden.

Geeignete Fettsäureester, die mit den N-Alkylglucaminen zu N-Alkyl-N-Acylglucaminen umgesetzt werden, sind im Allgemeinen die Alkylester, speziell die entsprechenden Methylester oder Ethylester, die durch Umesterung aus natürlichen Fetten und Ölen, beispielsweise den Triglyceriden, gewonnen werden.

Geeignete Rohstoffe für die Herstellung der Fettsäurealkylester sind beispielsweise Kokosöl oder Palmöl, wobei Kokosöl besonders bevorzugt ist.

Als weitere Komponente enthält eine erfindungsgemäße Zusammensetzung mindestens eine Fettsäure und/oder Seife als Komponente B.

Die Fettsäuren der Komponente B sind bevorzugt natürliche Fettsäuren, besonders bevorzugt mit 8 bis 22 C-Atomen, wie beispielsweise Octansäure, Decansäure, Laurinsäure, Myristinsäure, Palmitinsäure, Stearinsäure, Ölsäure, Linolsäure, Linolensäure, Behensäure, Hydroxyfettsäuren, beispielsweise 12-Hydroxystearinsäure oder 16-Hydroxyhexadecanoylsäure und deren Mischungen.

Besonders bevorzugt sind Fettsäuren mit 12 bis 18 C-Atomen.

Die Seifen der Komponente B sind Salze der Fettsäuren, insbesondere Alkalisalze, vorzugweise Natrium- oder Kaliumsalze. Die Fettsäuren weisen dabei insbesondere 8 bis 22 C-Atome, vorzugsweise 12 bis 18 C-Atome auf. Beispiele für Seifen sind Natriumstearat, Natriumpalmitat, Natriumlaurat, Natriummyristat, Natriumbehenat, Kaliumstearat, Kaliumpalmitat, Natriummyristat, Natriumoleat, Salze von Hydroxyfettsäuren, beispielsweise Salze von 12-Hydroxystearinsäure oder Salze von 16-Hydroxyhexadecanoylsäure.

Bevorzugt als Komponente B sind Laurinsäure, Palmitinsäure, Stearinsäure sowie deren Mischungen und Salze. Die Fettsäuren werden im Allgemeinen verwendet, um der Zusammensetzung ein rückfettendes und pflegendes Hautgefühl zu verleihen.

Eine erfindungsgemäße Zusammensetzung kann bevorzugt einen Anteil an Komponente B von 25,0-50,0 Gew.%, bezogen auf die Zusammensetzung, und besonders bevorzugt von 25,0-35,0 Gew.%, bezogen auf die Zusammensetzung, aufweisen.
Eine erfindungsgemäße Zusammensetzung umfasst mindestens ein Acylisethionat als Komponente C.

Im Rahmen einer bevorzugten Ausführungsform umfasst die erfindungsgemäße Zusammensetzung mindestens ein Acylisethionat der Formel (II) als Komponente C:

R-CO-O-CHR¹-CHR²-SO₃X (II)

worin
R den Alkylrest einer C₈-C₁₈-Fettsäure bedeutet,
R¹ und R² unabhängig voneinander H oder CH₃, vorzugsweise H, bedeuten und
X ein Kation, vorzugsweise ein Alkalimetallkation, insbesondere Na, ist.

Hierunter fallen Acylisethionate und Methyl-acylisethionate mit einem C₈-C₁₈ Acylrest und deren Mischungen, bevorzugt deren Natriumsalze. Besonders bevorzugt sind Natriumlauroylisethionat oder Natriumcocoylisethionate.

Durch die Zugabe der Komponente C zeigt eine erfindungsgemäße Zusammensetzung eine besonders gute Stabilität.

Eine erfindungsgemäße Zusammensetzung kann bevorzugt einen Anteil an Komponente C von 28,0-50,0 Gew.%, bezogen auf die Zusammensetzung und besonders bevorzugt von 40,0-48,0 Gew.% bezogen auf die Zusammensetzung aufweisen.

Ferner enthält eine erfindungsgemäße Zusammensetzung Natriumisethionat, das Natriumsalz der 2-Hydroxyethansulfonsäure, als Komponente D.
Eine erfindungsgemäße Zusammensetzung kann bevorzugt einen Anteil an Komponente D von 2,0-10,0 Gew.%, bezogen auf die Zusammensetzung, und besonders bevorzugt von 3,0-6,0 Gew.%, bezogen auf die Zusammensetzung, aufweisen.

Durch die Zugabe von Natriumisethionat zu der erfindungsgemäßen Zusammensetzung kann beispielsweise die Körnigkeit ("Grittiness") der Zusammensetzung eingestellt werden.

Ein wichtiges Kriterium bei der Beurteilung von Seifenstücken ist die Frage, ob sich die Seife bei der Anwendung auf der Haut weich anfühlt, oder rau. Die Rauigkeit hängt von der Körnigkeit der Seife ab und kann durch den Einsatz von Natriumisethionat eingestellt werden. Die Zugabe von Natriumisethionat, insbesondere in der hier beschriebenen Menge, kann in einer erfindungsgemäßen Zusammensetzung zu einer Verbesserung der Körnigkeit führen und damit zu einer Verringerung der Rauigkeit.

Eine erfindungsgemäße Zusammensetzung enthält ferner Wasser als Komponente E.

Vorzugsweise weist eine erfindungsgemäße Zusammensetzung einen Wassergehalt von 1,0-10,0 Gew.%, besonders bevorzugt von 2,0-7,0 Gew.%, bezogen auf die Zusammensetzung, auf.

In einer Ausführungsform enthält die erfindungsgemäße Zusammensetzung ein oder mehrere Additive F, bevorzugt aus der Gruppe bestehend aus Konservierungsmitteln, Duftstoffen, Farbstoffen, weiteren Tensiden, kationischen Polymeren, Pigmenten, Überfettungsmitteln, antimikrobiellen und biogenen Wirkstoffen, feuchtigkeitsspendenden Mitteln, Stabilisatoren, Säuren, Laugen, und Mischungen daraus, bevorzugt in Mengen von 1,0-20,0 Gew.-%, besonders bevorzugt von 2,0-15,0 Gew.-% und insbesondere von 3,0-10,0 Gew.-% jeweils bezogen auf die gesamte Zusammensetzung.
Als Konservierungsmittel eignen sich alle im betreffenden Annex der europäischen Kosmetikgesetzgebung gelisteten Konservierungsmittel, beispielsweise Phenoxyethanol, Benzylalkohol, Parabene, Benzoesäure und Sorbinsäure, besonders gut geeignet ist beispielsweise 1,3-Bis(hydroxymethyl)-5,5-dimethylimidazolidine-2,4-dione (Nipaguard® DMDMH), Pirocton Olamine, Methylisothiazolinon oder Mischungen daraus, bevorzugt Pirocton Olamin und/oder Methylisothiazolinon.

Als Duft- bzw. Parfümstoffe oder Öle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die Ionone, alpha-Isomethylionon und Methyl-cedrylketon, zu den Alkoholen Anethol, Citronellol, Eugenol, Geraniol, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als Farbstoffe eignen sich im Prinzip alle Farbstoffe, die für den Kosmetikgebrauch zugelassen sind, diese sind in den entsprechenden Anhängen der europäischen Kosmetikgesetzgebung gelistet sind.
Beispielsweise kann es sich bei den enthaltenen Farbstoffen und Pigmenten, sowohl um organische als auch anorganische Farbstoffe handeln. Vorteilhaft eingesetzt werden auch Perlglanzpigmente, z.B. Fischsilber (Guanin/ Hypoxanthin-Mischkristalle aus Fischschuppen) und Perlmutt (vermahlene Muschelschalen), monokristalline Perlglanzpigmente wie z.B. Bismuthoxychlorid (BiOCI), Schicht-Substrat Pigmente, z.B. Glimmer/Metalloxid, silberweiße Perlglanzpigmente aus TiO₂, Interferenzpigmente (TiO₂, unterschiedliche Schichtdicke), Farbglanzpigmente (Fe₂O₃) und Kombinationspigmente (TiO₂/Fe₂O₃, TiO₂/Cr₂O₃, TiO₂/Berliner Blau, TiO₂/Carmin).

Die Menge der Farbstoffe und Pigmente in den erfindungsgemäßen Zusammensetzungen beträgt im Allgemeinen von 0,1-2,0 Gew.-%, bezogen auf das Gesamtgewicht der fertigen Zusammensetzungen.

Weitere Tenside können im Prinzip alle anionischen, kationischen oder amphoteren Tenside sein, die kosmetikgeeignet sind.

Geeignete anionische Tenside können ausgewählt sein aus der Gruppe der Alkylsulfate und Alkylethersulfate.

Bevorzugte Alkylsulfate sind die C₈-C₂₀-Alkylsulfate, insbesondere die linearen C₈-C₂₀-Alkylsulfate vorzugsweise in Form ihrer Natrium-, Kalium- oder Ammoniumsalze. Beispiele für Alkylsulfate sind Laurylsulfat, Cocosalkylsulfat und Talgalkylsulfat. Besonders bevorzugt ist Laurylsulfat.

Bevorzugte Alkylethersulfate sind die C₈-C₂₀-Alkylethersulfate, besonders bevorzugt sind die linearen C₈-C₂₀-Alkylethersulfate, insbesondere die von den ethoxylierten Fettalkoholen abgeleiteten Alkylglykolethersulfate, in Form ihrer Natrium-, Kalium- oder Ammoniumsalze. Beispiele für Alkylethersulfate sind Laurylethersulfat, Cocosalkylethersulfat und Talgalkylethersulfat. Besonders bevorzugt ist Laurylethersulfat. Beispiele für Glykolethersulfate sind Lauryltriethylenglykolethersulfat, Cocosalkyltriethylenglykolethersulfat und Talgalkylhexaethylenglykolethersulfat. Insbesondere bevorzugt ist Laurylglykolethersulfat, beispielsweise, Lauryldiethylenglykolethersulfat oder Lauryltriethylenglykolethersulfat, speziell in Form der Natriumsalze.

Ein besonders bevorzugtes anionisches Tensid ist Natriumlaurylethersulfat.

In einer weiteren Ausführungsform der Erfindung kann die Zusammensetzung ein oder mehrere N-Acylaminosäuretenside als anionische Tenside enthalten. Im Rahmen einer bevorzugten Ausführungsform ist der Aminosäurerest solcher N-Acyl-aminosäuretenside ausgewählt aus der Gruppe bestehend aus proteinogenen Aminosäuren, deren N-alkylierten Derivaten und Mischungen daraus.

Besonders bevorzugt als N-Acyl-aminosäuretenside sind Acylglycinate, Acylalaninate, Acylaspartate, Acylglutamate, Acylsarkosinate oder Mischungen davon. Ganz besonders bevorzugt sind die N-Acyl-aminosäuretenside ausgewählt aus der Gruppe bestehend aus Acylglycinat, Acylaspartat, Acylglutamat, Acylsarkosinat und Mischungen daraus.

Ganz besonders bevorzugt bestehen die N-Acyl-aminosäuretenside aus mindestens einer C₈-C₂₂-acylierten Aminosäure, insbesondere deren N-alkylierten Derivaten. Bevorzugt sind die entsprechenden Lauroyl - oder Cocoylderivate der Aminosäuren.

Insbesondere bevorzugt sind daher Natriumcocoylglycinat, Kaliumcocoylglycinat, Natriumlauroylglycinat, Kaliumlauroylglycinat, Natriumcocoylglutamat, Natriumlauroylglutamat, Natriumcocoylaspartat, Natriumlauroylaspartat und Natriumlauroylsarkosinat.

Geeignete kationische Tenside sind substituierte oder unsubstituierte geradkettige oder verzweigte quartäre Ammoniumsalze vom Typ R¹N(CH₃)₃X, R¹R²N(CH₃)₂X, R¹R²R³N(CH₃)X oder R¹R²R³R⁴NX. Die Reste R¹, R², R³ und R⁴ können vorzugsweise unabhängig voneinander unsubstituiertes Alkyl mit einer Kettenlänge zwischen 8 und 24 C-Atomen, insbesondere zwischen 10 und 18 C-Atomen, Hydroxyalkyl mit 1 bis 4 C-Atomen, Phenyl, C₂- bis C₁₈-Alkenyl, C₇- bis C₂₄-Aralkyl, (C₂H₄O)ₓH, wobei x von 1 bis 3 bedeutet, ein oder mehrere Estergruppen enthaltende Alkylreste oder cyclische quartäre Ammoniumsalze sein. X ist ein geeignetes Anion. Bevorzugt sind (C₈-C₂₂)-Alkyltrimethylammoniumchlorid oder -bromid, besonders bevorzugt Cetyltrimethylammoniumchlorid oder -bromid, Di-(C₈-C₂₂)-Alkyldimethylammoniumchlorid oder -bromid, (C₈-C₂₂)-Alkyldimethylbenzylammoniumchlorid oder -bromid, (C₈-C₂₂)-Alkyl-dimethylhydroxyethylammoniumchlorid, -phosphat, -sulfat, -lactat, besonders bevorzugt Distearyldimethylammoniumchlorid, Di(C₈-C₂₂)-Alkylamidopropyltrimethylammoniumchlorid und -methosulfat.

Als nichtionische Tenside kommen beispielsweise folgende Verbindungen in Frage:
Polyethylen-, Polypropylen- und Polybutylenoxidkondensate von Alkylphenolen. Diese Verbindungen umfassen die Kondensationsprodukte von Alkylphenolen mit einer C₆- bis C₂₀-Alkylgruppe, die entweder linear oder verzweigt sein kann, sowie mit Alkenoxiden. Diese Tenside werden als Alkylphenolalkoxylate, z.B. Alkylphenolethoxylate, bezeichnet.

Kondensationsprodukte von aliphatischen Alkoholen mit 1 bis 25 mol Ethylenoxid. Die Alkyl- oder Alkenylkette der aliphatischen Alkohole kann linear oder verzweigt, primär oder sekundär sein, und enthält im Allgemeinen 8 bis 22 Kohlenstoffatome. Besonders bevorzugt sind die Kondensationsprodukte von C₁₀- bis C₂₀-Alkoholen mit 2 bis 18 mol Ethylenoxid pro mol Alkohol. Die Alkoholethoxylate können eine enge ("Narrow Range Ethoxylates") oder eine breite Homologenverteilung des Ethylenoxides ("Broad Range Ethoxylates") aufweisen. Beispiele von kommerziell erhältlichen nichtionischen Tensiden dieses Typs sind Tergitol® 15-S-9 (Kondensationsprodukt eines linearen sekundären C₁₁-C₁₅-Alkohols mit 9 mol Ethylenoxid), Tergitol® 24-L-NMW (Kondensationsprodukt eines linearen primären C₁₂-C₁₄-Alkohols mit 6 mol Ethylenoxid bei enger Molgewichtsverteilung). Ebenfalls unter diese Produktklasse fallen die Genapol®-Marken der Clariant. Kondensationsprodukte von Ethylenoxid mit einer hydrophoben Basis, gebildet durch Kondensation von Propylenoxid mit Propylenglykol. Der hydrophobe Teil dieser Verbindungen weist bevorzugt ein Molekulargewicht zwischen 1500 und 1800 auf. Die Anlagerung von Ethylenoxid an diesen hydrophoben Teil führt zu einer Verbesserung der Wasserlöslichkeit. Das Produkt ist flüssig bis zu einem Polyoxyethylengehalt von ca. 50 % des Gesamtgewichtes des Kondensationsproduktes, was einer Kondensation mit bis zu ca. 40 mol Ethylenoxid entspricht. Kommerziell erhältliche Beispiele dieser Produktklasse sind die Pluronic®-Marken der BASF und die Genapol® PF-Marken der Clariant.

Kondensationsprodukte von Ethylenoxid mit einem Reaktionsprodukt von Propylenoxid und Ethylendiamin. Die hydrophobe Einheit dieser Verbindungen besteht aus dem Reaktionsprodukt von Ethylendiamin mit überschüssigem Propylenoxid und weist im Allgemeinen ein Molekulargewicht von 2500 bis 3000 auf. An diese hydrophobe Einheit wird Ethylenoxid bis zu einem Gehalt von 40 bis 80 Gew.-% Polyoxyethylen und einem Molekulargewicht von 5000 bis 11000 addiert. Kommerziell erhältliche Beispiele dieser Verbindungsklasse sind die Tetronic®-Marken der BASF und die Genapol® PN-Marken der Clariant.

Als nichtionische Tenside bevorzugt sind Fettalkoholethoxylate (Alkylpolyethylenglykole); Alkylphenolpolyethylenglykole; Fettaminethoxylate (Alkylaminopolyethylenglykole); Fettsäureethoxylate (Acylpolyethylenglykole); Polypropylenglykolethoxylate (Pluronics®); Fettsäurealkanolamide, (Fettsäureamidpolyethylenglykole); Saccharoseester; Sorbitester und Sorbitanester und deren Polyglykolether, sowie C₈-C₂₂-Alkylpolyglucoside.

Weiterhin können die erfindungsgemäßen Zusammensetzungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z.B. mit einer Carboxyl-, Sulfat- oder Sulfonat-Gruppe. Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze. Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die C₁₀- bis C₁₈-Alkyldimethylaminoxide und Fettsäureamidoalkyl-dimethylaminoxide.

In einer bevorzugten Ausführungsform enthalten die erfindungsgemäßen Zusammensetzungen zusätzlich noch als schaumverstärkende Mittel Cotenside aus der Gruppe der Alkylamidobetaine, Aminopropionate, Aminoglycinate, Imidazoliniumbetaine und Sulfobetaine, Aminoxide, Fettsäurealkanolamide und Polyhydroxyamide.

Bevorzugt als weitere Tenside sind: Ethoxylierte und propoxylierte Fettalkohole, ethoxylierte und propoxylierte Triglyceride wie PEG-40 hydrogenated Castor Oil oder Fettsäureester, Ethercarboxylate, Alkylpolyglucoside, Olefinsulfonate, sec. Alkylsulfonate und Taurate.

Als kationische Polymere eignen sich die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVPdimethylaminoethylmethacrylat-Copolymer, Guarhydroxypropyltriammoniumchloride, sowie Calciumalginat und Ammoniumalginat. Des Weiteren können eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxypropyldiethylentriamin; Polyaminopolyamid und kationische Chitinderivate, wie beispielsweise Chitosan.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, sowie ethoxylierte Triglyceride wie PEG-7 Glyceryl Cocoat oder Mischungen aus Glyceryloleat mit Alkylpolyglucosiden Verwendung finden.

An antimikrobiellen Wirkstoffen kommen Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox, lodopropynylbutylcarbamat, Methylchloroisothiazolinon, Methylisothiazolinon, Methyldibromo Glutaronitril, AgCI, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, Pentandiol, 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat, Hydroxyethylglycin der Sorbinsäure und Kombinationen dieser Wirksubstanzen zum Einsatz.

Die erfindungsgemäßen Zusammensetzungen können des Weiteren biogene Wirkstoffe ausgewählt aus Pflanzenextrakten, wie beispielsweise Aloe Vera, sowie Lokalanästhetika, Antibiotika, Antiphlogistika, Antiallergica, Corticosteroide, Sebostatika, Bisabolol®, Allantoin®, Phytantriol®, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Als feuchtigkeitsspendende Substanz stehen beispielsweise Isopropylpalmitat, Glycerin, Diglycerin und/oder Sorbitol zu Verfügung. Besonders bevorzugt ist Glycerin.

Bevorzugt weist eine erfindungsgemäße Zusammensetzung einen pH-Wert von 3 bis 9, besonders bevorzugt von 5 bis 8, auf.

Als Säuren oder Laugen zur pH-Wert Einstellung werden vorzugsweise Mineralsäuren, insbesondere HCl, anorganische Basen, insbesondere NaOH oder KOH, oder organische Säuren, insbesondere Zitronensäure oder Milchsäure, verwendet.

Eine erfindungsgemäße Zusammensetzung kann auch Komplexierungsmittel aufweisen, durch die beispielsweise Alkaliionen komplexiert werden können und so die Stabilität der Zusammensetzung verbessert werden kann. Typische Komplexierungsmittel sind zum Beispiel EDTA (Ethylendiamin-tetraacetat) und Nitrilotriessigsäure. Bevorzugt wird EDTA als Komplexierungsmittel eingesetzt.

Eine erfindungsgemäße Zusammensetzung kann neben Wasser auch mindestens ein weiteres Lösungsmittel enthalten. Unter einem Lösungsmittel im Rahmen der vorliegenden Erfindung versteht man vorzugsweise protische Lösungsmittel wie C₁-C₈-Alkohole, insbesondere C₁-C₆-Alkohole, Ethylenglykol, Diethylenglykol, Triethylenglykol oder Mischungen davon, wobei insbesondere Wasser und/oder Ethanol oder Wasser und/oder Methanol bevorzugt sind. Von den C₁-C₆-Alkoholen sind Methanol, Ethanol, Isopropanol, n-Butanol oder sek.-Butanol bevorzugt.

Des Weiteren können die erfindungsgemäßen Zusammensetzungen Filmbildner enthalten, die je nach Anwendungszweck ausgewählt sind aus Salzen der Phenylbenzimidazolsulfonsäure, wasserlöslichen Polyurethanen, beispielsweise C₁₀-Polycarbamylpolyglycerylester, Polyvinylalkohol, Polyvinylpyrrolidoncopolymeren wie PVP/Hexandecene oder PVP/Eicosene Copolymer, beispielsweise Vinylpyrrolidon/Vinylacetatcopolymer, wasserlöslichen Acrylsäurepolymeren/Copolymeren bzw. deren Estern oder Salzen, beispielsweise Partialestercopolymere der Acryl/Methacrylsäure und Polyethylenglykolethern von Fettalkoholen, wie Acrylat/Steareth-20-Methacrylat Copolymer, wasserlöslicher Cellulose, beispielsweise Hydroxymethylcellulose, Hydroxyethylcellulose, Hydroxypropylcellulose, wasserlöslichen Quaterniums, Polyquaterniums, Carboxyvinyl-Polymeren, wie Carbomere und deren Salze, Polysacchariden, beispielsweise Polydextrose und Glucan, Vinylacetat/Crotonat, beispielsweise unter dem Handelsnamen Aristoflex® A 60 (Clariant) erhältlich, sowie polymeren Aminoxiden, beispielsweise unter den Handelsnamen Diaformer Z-711, 712, 731, 751 erhältliche Vertreter.

Eine erfindungsgemäße Zusammensetzung kann auch mindestens einen Ölkörper aufweisen. Die Ölkörper können vorteilhafterweise ausgewählt werden aus den Gruppen der natürlichen und synthetische Fettkörper, vorzugsweise Triglyceride, Ester von Fettsäuren mit Alkoholen niedriger C-Zahl, z. B. mit Isopropanol, Propylenglycol oder Glycerin, oder Ester von Fettalkoholen mit Alkansäuren niedriger C-Zahl oder mit Fettsäuren oder aus der Gruppe der Alkylbenzoate, sowie natürliche oder synthetische Kohlenwasserstofföle und Silikonöle.

Besonders bevorzugt sind Triglyceridöle wie Sonnenblumen- und Sojaöl; ebenso besonders bevorzugt ist Petrolatum (Vaseline).

In Betracht kommen bevorzugt Triglyceride von linearen oder verzweigten, gesättigten oder ungesättigten, gegebenenfalls hydroxylierten, C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z. B. das Handelsprodukt Myritol® 318. Auch gehärtete Triglyceride sind erfindungsgemäß bevorzugt. Auch Öle tierischen Ursprungs, beispielweise Rindertalg, Perhydrosqualen, Lanolin können eingesetzt werden.

Weiterhin erfindungsgemäß bevorzugte Ölkörpern sind die Benzoesäureester von linearen oder verzweigten C₈₋₂₂-Alkanolen, z. B. die Handelsprodukte Finsolv® SB (Isostearylbenzoat), Finsolv® TN (C₁₂-C₁₅-Alkylbenzoat) und Finsolv® EB (Ethylhexylbenzoat).

Eine weitere Klasse von erfindungsgemäß bevorzugten Ölkörper sind Dialkylether mit insgesamt 12 bis 36 Kohlenstoffatomen, insbesondere mit 12 bis 24 Kohlenstoffatomen, wie z. B. Di-n-octylether (Cetiol® OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether, Di-3-ethyldecylether, tert.-Butyl-n-octylether, iso-Pentyl-n-octylether und 2-Methylpentyl-n-octylether sowie Di-tert.-butylether und Di-iso-pentylether.

Ebenso in Betracht kommen verzweigte gesättigte oder ungesättigte Fettalkohole mit 6 - 30 Kohlenstoffatomen, z. B. Isostearylalkohol, sowie Guerbetalkohole.

Eine weitere Klasse von bevorzugten Ölkörpern sind Dicarbonsäureester von linearen oder verzweigten C₂-C₁₀-Alkanolen, wie Di-n-butyladipat (Cetiol® B), Di-(2-ethylhexyl)-adipat und Di-(2-ethylhexyl)-succinat sowie Diolester wie Ethylenglykoldioleat, Ethylenglykoldiisotridecanoat, Propylenglycol-di-(2-ethylhexanoat), Propylenglycoldiisostearat, Propylenglycol-di-pelargonat, Butandiol-di-isostearat und Neopentylglycoldicaprylat sowie Diisotridecylacetat.

Ebenso bevorzugte Ölkörper sind symmetrische, unsymmetrische oder cyclische Ester der Kohlensäure mit Fettalkoholen, Glycerincarbonat oder Dicaprylylcarbonat (Cetiol® CC).

Eine weitere Klasse von bevorzugten Ölkörpern sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertig linearen oder verzweigten C₂-C₆-Alkanolen.

Eine weitere Klasse von bevorzugten Ölkörpern sind Kohlenwasserstofföle, zum Beispiel solche mit linearen oder verzweigten, gesättigten oder ungesättigten C₇-C₄₀-Kohtenstoffketten, beispielsweise Vaseline, Dodecan, Isododecan, Cholesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z. B. die Handelsprodukte der Permethyl®-Serie, Squalan, Squalen, und alicyclische Kohlenwasserstoffe, z. B. das Handelsprodukt 1,3-Di-(2-ethyl-hexyl)-cyclohexan (Cetiol® S), Ozokerit und Ceresin.

Bevorzugte erfindungsgemäße Seifenstücke enthalten:
- mindestens ein N-Alkyl-N-Acylglucamin, wobei das N-Alkyl-N-Acylglucamin mindestens eine C₁₂- und/oder C₁₄-Acylgruppe enthält,
- mindestens eine Fettsäure und/oder Seife, wobei es sich bei der Fettsäure vorzugsweise um Stearinsäure handelt,
- Natriumcocoylisethionat oder Natriumlauroylisethionat als Komponente C und
- Natriumisethionat als Komponente D,
- wobei mehr als 70 Gew.% der N-Alkyl-N-Acylglucamine mindestens eine C₁₂- und/oder C₁₄-Acylgruppe enthalten.
Die bevorzugte Ausführungsform kann sowohl als Combibar oder Syndetbar eingesetzt werden.

Weiter bevorzugte Seifenstücke Zusammensetzungen enthalten:
- mindestens ein N-Alkyl-N-Acylglucamin, wobei das N-Alkyl-N-Acylglucamin von Kokosöl abgeleitet ist,
- mindestens eine Fettsäure und/oder Seife, wobei es sich bei der Fettsäure vorzugsweise um Stearinsäure handelt,
- Natriumcocoylisethionat als Komponente C und
- Natriumisethionat als Komponente D,
wobei mehr als 70 Gew.% der N-Alkyl-N-Acylglucamine mindestens eine C₁₂- und/oder C₁₄-Acylgruppe enthalten.

Die bevorzugten Seifenstücke können sowohl als Combibar oder Syndetbar eingesetzt werden.

Im Rahmen einer bevorzugten Ausführungsform handelt es sich bei der erfindungsgemäßen Zusammensetzung um eine kosmetische oder dermatologische Zusammensetzung.

Ein weiterer Gegenstand der Erfindung ist die Verwendung des erfindungsgemäßen Seifenstücks als Combibar oder Syndetbar.

Ein weiterer Gegenstand der Erfindung ist die Verwendung des erfindungsgemäßen Seifenstücks zur Behandlung oder Pflege der Haut.

Ein weiterer Gegenstand der Erfindung ist die Verwendung des erfindungsgemäßen Seifenstücks zur Behandlung oder Pflege der Haare.

Ferner ist Gegenstand der Erfindung ein Verfahren zur Herstellung eines Seifenstücks, wobei die Komponenten A, B, C, D, E und gegebenenfalls F miteinander in Kontakt gebracht werden. Die Herstellung kann nach gängigen Methoden erfolgen. Dabei werden die Komponenten des erfindungsgemäßen Seifenstücks miteinander vermischt, was z.B. durch Kneten erfolgen kann. Die erhaltene Masse kann z.B. durch Extrudieren, Schneiden, Stückpressen oder Gießen in die gewünschte Form gebracht werden. Die Herstellung erfolgt vorzugsweise bei erhöhten Temperaturen, insbesondere bei Temperaturen zwischen 40°C und 90°C.
Die nachfolgenden Beispiele dienen der Erläuterung der Erfindung, ohne sie jedoch darauf einzuschränken.

### Beispiele

Die im Folgenden beschriebenen N-Alkyl-N-Acylglucamine wurden nach EP 0 550 637 aus den korrespondierenden Fettsäuremethylestern und N-Acylglucamid in Gegenwart von 1,2 Propylenglykol als Lösemittel hergestellt und als Feststoff bestehend aus Aktivsubstanz, d.h. N-Alkyl-N-Acylglucamin, und 1,2 Propylenglykol erhalten (alle Angaben in Gew.-%).

**Tabelle 1: Herstellbeispiele für N-Alkyl-N-Acylglucamin**

| Herstellbeispiel | Methylester | Triglycerid | Aktivsubstanz (%) | 1,2-Propylenglykol (%) | Schmelzpunkt (°C) |
|---|---|---|---|---|---|
| 1 | C12/14 | - | 90 | 10 | 85 |
| | (C12: 70 %, C14 30 %) | | | | |
| 2 | C 16/18 | - | 80 | 20 | 65 |
| | (C16: 60 %; C 18: 40 %) | | | | |
| 3 | - | Kokosöl | 90 | 10 | 55 |
| | | (C8: 6 % C10: 6 % C12: 48 % C14: 20% C18: 2 % C18': 8 %) | | | |

C18' bedeutet eine Oleoylrest.

Der Schmelzpunkt wurde mittels einer Kofler-Heizbank bestimmt.

### Zusammensetzungen (Seifenstücke) (alle Prozentzahlen sind Gew.% bezogen auf die Zusammensetzung)

### Zusammensetzung Nr. 1 (Anwendung als Syndetbar)

46,2% Hostapon SCI 65C (enthält 65% Natriumcocoylisethionat und 35% Stearinsäure)
25,2% Stearinsäure
1% Glucamin (gemäß Tabelle 1)
10% Polyglycol 20000P
5% Hostapon SI (57% Lösung von Natriumisethionat in Wasser)
0,05% Etidronsäure
2,0% Maisstärke
0,3% Titandioxid
9,25% Wasser (entionisiert)
1% Zitronensäure

### Zusammensetzung Nr. 2 (Standard für Syndet-Seife)

47,2% Hostapon SCI 65C (enthält 65% Natriumcocoylisethionat und 35% Stearinsäure)
25,2% Stearinsäure
0% N-Alkyl-N-Acylglucamin (gemäß Tabelle 1)
10% Polyglycol 20000P
5% Hostapon SI (57% Lösung von Natriumisethionat in Wasser)
0,05% Etidronsäure
2,0% Maisstärke
0,3% Titandioxid
9,25% Wasser (entionisiert)
1% Zitronensäure

### Herstellung der Zusammensetzungen

Die Zusammensetzungen wurden in einer Seifenmaschine, die aus einem Laborkneter (Typ LTK 3R), Walzenflocker (W3K-32 R) und einem Schneckenextruder (SVZ 75R) besteht, hergestellt.

**Tabelle 2: Eigenschaften der Zusammensetzungen im Vergleich zur Zusammensetzung 2**

| Nr. der Seife | Zusammensetzung | Schaummenge | Härte |
|---|---|---|---|
| 1 | Zusammensetzung 1 | mehr Schaum | brüchig |
| | (N-Alkyl-N-Acylglucamin gemäß Herstellbeispiel 2) | | |
| 2 | Zusammensetzung 1 | mehr Schaum | erhöhte Härte |
| | (N-Alkyl-N-Acylglucamin gemäß Herstellbeispiel 3) | | |
| 3 | Zusammensetzung 1 | mehr Schaum | erhöhte Härte |
| | (N-Alkyl-N-Acylglucamin gemäß Herstellbeispiel 1) | | |

Die Eigenschaften wurden durch drei Testpersonen durch Händewaschen mit den Zusammensetzungen ermittelt. Die Härte wurde durch manuelle Evaluierung ermittelt.

Die Schaummenge als auch die Härte wurden im Vergleich zu Zusammensetzung 2 bestimmt, die keine N-Alkyl-N-Acylglucamine enthalten.

### Ergebnis

N-Alkyl-N-Acylglucamine gemäß Herstellbeispiele 1 und 3 erhöhen in der Zusammensetzung 1 die Schaummenge und führen zu einer erhöhten Härte im Vergleich zum Standard (Zusammensetzung 2).

### Analyse des Auflöseverhaltens ("Mushiness") der Zusammensetzungen als Seifenstücke

### Durchführung der Analyse

Jedes Seifenstück wird in ein Gefäß mit gleichem Volumen gelegt. Dabei wird die Position der Seifenstücke so gut es geht ähnlich gehalten. Nach Positionierung des Seifenstückes wird das Gefäß mit einer bestimmten Menge Wasser gefüllt, die für jedes Gefäß und damit für jedes Seifenstück identisch ist. Jedes Gefäß wird mit Alufolie verschlossen, um ein Abdampfen zu vermeiden und bei Raumtemperatur gelagert. Nach 1, 4, 5, 9 und 14 Tagen wird die Länge jedes Seifenstücks gemessen. Die Ergebnisse sind in Tabelle 3 dargestellt.

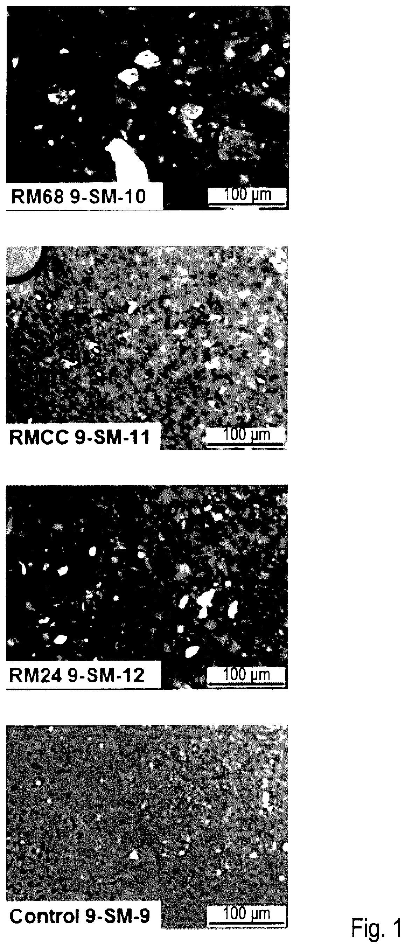

Zur Bestimmung des Mechanismus der zum Auflösen ("Mushiness") der Seifenstücke führt, wird nach einem Tag die Oberfläche des Seifenstücks mittels Mikroskopie unter Verwendung von polarisiertem Licht analysiert. Die Ergebnisse sind in Figur 1 dargestellt.

Die erfindungsgemäßen Seifen 1, 2 und 3 mit einer lamellaren Struktur der Oberfläche weisen dabei eine langsamere Auflösung in Wasser auf, was in der Praxis zu einer längeren Haltbarkeit der Syndetseife beim Verbraucher führt. Die Kontrollprobe (Zusammensetzung 2, Control 9-SM-9) ist dagegen bereits nach einem Tag deutlich geschrumpft.

**Tabelle 3: Angabe der Länge des Seifenstücks in [cm] in Abhängigkeit von der Zusammensetzung und Anzahl der Tage. Die Seifen 1, 2 und 3 entsprechen denen aus Tablle 2.**

| Seife Nr. | 0 Tage | 1 Tag | 4 Tage | 5 Tage | 9 Tage | 14 Tage |
|---|---|---|---|---|---|---|
| 1 (RM68 9-SM-10) | 6,0 | 5,5 | 3,5 | 0 | | |
| 2 (RMCC 9-SM-11) | 5,5 | 5,0 | 0 | | | |
| 3 (RM24 9-SM-12) | 6,0 | 5,5 | 5,0 | 4,2 | 4,0 | 0 |
| Zusammensetzung 2 (Control 9-SM-9) | 6,0 | 4,5 | 0 | | | |

In Seifen der Zusammensetzung 1 verlangsamen insbesondere N-Alkyl-N-Acylglucamine gemäß Herstellbeispiel 1 mit einem C_{12/14} Kettenschnitt deutlich die Auflösung der Syndetseife.

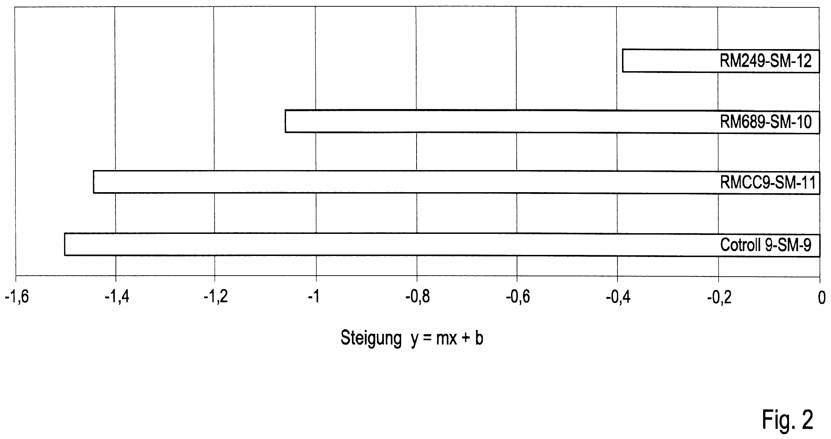

Die Steigungen der Ergebnisse aus Tabelle 3 sind in Figur 2 dargestellt.

## Patentansprüche

1. Seifenstück enthaltend:
- mindestens ein N-Alkyl-N-Acylglucamin als Komponente A,
- mindestens eine Fettsäure und/oder Seife als Komponente B,
- mindestens ein Acylisethionat als Komponente C,
- Natriumisethionat als Komponente D,
- Wasser als Komponente E,
- optional mindestens ein weiteres Additiv als Komponente F,
wobei mehr als 20 Gew.%, vorzugsweise mehr als 70 Gew.%, der N-Alkyl-N-Acylglucamine mindestens eine C₁₂- und/oder C₁₄- und/oder C₁₆- und/oder C₁₈-Acylgruppe enthalten.

2. Seifenstück nach Anspruch 1, enthaltend:
- 0,5-5,0 Gew.%, vorzugsweise 1,0-3,0 Gew.%, der Zusammensetzung an Komponente A,
- 25,0-50,0 Gew.%, vorzugsweise 25,0-35,0 Gew.%, der Zusammensetzung an Komponente B,
- 28,0-50,0 Gew.%, vorzugsweise 40,0-48,0 Gew.%, der Zusammensetzung an Komponente C,
- 2,0-10,0 Gew.%, vorzugsweise 3,0-6,0 Gew.%, der Zusammensetzung an Komponente D.

3. Seifenstück nach einem der Ansprüche 1 oder 2, wobei mehr als 90 Gew.% der N-Alkyl-N-Acylglucamine mindestens eine C₁₂- und/oder C₁₄- und/oder C₁₆- und/oder C₁₈-Acylgruppe enthalten.

4. Seifenstück nach einem der Ansprüche 1 bis 3, wobei die Komponente A aus einem oder mehreren C₈-C₂₂ N-Alkyl-N-Acylglucaminen besteht.

5. Seifenstück nach einem der Ansprüche 1 bis 4, wobei die Komponente A aus einem oder mehreren C₈-C₂₂-N-Methyl-N-Acylglucaminen besteht.

6. Seifenstück nach einem der Ansprüche 1 bis 5, wobei mindestens eine Komponente B eine C₁₂-C₁₈ Fettsäure, deren Salz oder eine Mischung daraus enthält.

7. Seifenstück nach einem der Ansprüche 1 bis 6, wobei mindestens eine Komponente B aus Laurinsäure, Palmitinsäure, Stearinsäure, deren Salze oder Mischungen daraus besteht.

8. Seifenstück nach einem der Ansprüche 1 bis 7, wobei mindestens eine Komponente C aus Natriumlauroylisethionat, Natriumcocoylisethionat oder deren Mischung besteht.

9. Seifenstück nach einem der Ansprüche 1 bis 8, wobei die mindestens eine Komponente F ausgewählt ist aus der Gruppe bestehend aus Konservierungsmitteln, Duftstoffen, Farbstoffen, Tensiden, kationischen Polymeren, Pigmenten, Überfettungsmitteln, antimikrobiellen und biogenen Wirkstoffen, feuchtigkeitsspendenden Mitteln, Stabilisatoren, Säuren und Laugen.

10. Seifenstück nach einem der Ansprüche 1 bis 9, wobei es sich um eine kosmetische oder dermatologische Zusammensetzung handelt.

11. Verwendung des Seifenstücks nach einem der Ansprüche 1 bis 10 als Combibar oder Syndetbar.

12. Verwendung des Seifenstücks nach einem der Ansprüche 1 bis 10 zur Behandlung oder Pflege der Haut, der Haare oder von Haut und Haaren.

13. Verfahren zur Herstellung eines Seifenstück nach einem der Ansprüche 1 bis 10, wobei die Komponenten A, B, C, D, E und gegebenenfalls F

## Claims

1. A soap bar comprising:
- at least one N-alkyl-N-acylglucamine as component A,
- at least one fatty acid and/or soap as component B,
- at least one acyl isethionate as component C,
- sodium isethionate as component D,
- water as component E,
- optionally at least one further additive as component F,
where more than 20% by weight, preferably more than 70% by weight, of the N-alkyl-N-acylglucamines contain at least one C₁₂- and/or C₁₄- and/or C₁₆- and/or C₁₈-acyl group.

2. The soap bar as claimed in claim 1, comprising:
- 0.5-5.0% by weight, preferably 1.0-3.0% by weight, of the composition of component A,
- 25.0-50.0% by weight, preferably 25.0-35.0% by weight, of the composition of component B,
- 28.0-50.0% by weight, preferably 40.0-48.0% by weight, of the composition of component C,
- 2.0-10.0% by weight, preferably 3.0-6.0% by weight, of the composition of component D.

3. The soap bar as claimed in one of claims 1 and 2, where more than 90% by weight of the N-alkyl-N-acylglucamines contain at least one C₁₂- and/or C₁₄- and/or C₁₆- and/or C₁₈-acyl group.

4. The soap bar as claimed in one of claims 1 to 3, where the component A consists of one or more C₈-C₂₂-N-alkyl-N-acylglucamines.

5. The soap bar as claimed in one of claims 1 to 4, where the component A consists of one or more C₈-C₂₂-N-methyl-N-acylglucamines.

6. The soap bar as claimed in one of claims 1 to 5, where at least one component B comprises a C₁₂-C₁₈ fatty acid, salt thereof or a mixture thereof.

7. The soap bar as claimed in one of claims 1 to 6, where at least one component B consists of lauric acid, palmitic acid, stearic acid, salts thereof or mixtures thereof.

8. The soap bar as claimed in one of claims 1 to 7, where at least one component C consists of sodium lauroyl isethionate, sodium cocoyl isethionate or mixture thereof.

9. The soap bar as claimed in one of claims 1 to 8, where the at least one component F is selected from the group consisting of preservatives, fragrances, dyes, surfactants, cationic polymers, pigments, superfatting agents, antimicrobial and biogenic active ingredients, moisturizing agents, stabilizers, acids and alkalis.

10. The soap bar as claimed in one of claims 1 to 9, where it is a cosmetic or dermatological composition.

11. The use of the soap bar as claimed in one of claims 1 to 10 as combibar or syndet bar.

12. The use of the soap bar as claimed in one of claims 1 to 10 for treating or caring for the skin, the hair or skin and hair.

13. A process for producing a soap bar as claimed in one of claims 1 to 10, where the components A, B, C, D, E and optionally F.

## Revendications

1. Bloc de savon contenant :
- au moins une N-alkyl-N-acylglucamine en tant que composant A,
- au moins un acide gras et/ou un savon en tant que composant B,
- au moins un iséthionate d'acyle en tant que composant C,
- de l'iséthionate de sodium en tant que composant D,
- de l'eau en tant que composant E,
- éventuellement au moins un additif supplémentaire en tant que composant F,
plus de 20 % en poids, de préférence plus de 70 % en poids, des N-alkyl-N-acylglucamines contenant au moins un groupe acyle en C₁₂ et/ou C₁₄ et/ou C₁₆ et/ou C₁₈.

2. Bloc de savon selon la revendication 1, contenant :
- 0,5 à 5,0 % en poids, de préférence 1,0 à 3,0 % en poids, de la composition de composant A,
- 25,0 à 50,0 % en poids, de préférence 25,0 à 35,0 % en poids, de la composition de composant B,
- 28,0 à 50,0 % en poids, de préférence 40,0 à 48,0 % en poids, de la composition de composant C,
- 2,0 à 10,0 % en poids, de préférence 3,0 à 6,0 % en poids, de la composition de composant D.

3. Bloc de savon selon l'une quelconque des revendications 1 ou 2, dans lequel plus de 90 % en poids des N-alkyl-N-acylglucamines contiennent au moins un groupe acyle en C₁₂ et/ou C₁₄ et/ou C₁₆ et/ou C₁₈.

4. Bloc de savon selon l'une quelconque des revendications 1 à 3, dans lequel le composant A est constitué par une ou plusieurs N-alkyl-N-acylglucamines en C₈-C₂₂.

5. Bloc de savon selon l'une quelconque des revendications 1 à 4, dans lequel le composant A est constitué par une ou plusieurs N-méthyl-N-acylglucamines en C₈-C₂₂.

6. Bloc de savon selon l'une quelconque des revendications 1 à 5, dans lequel au moins un composant B contient un acide gras en C₁₂-C₁₈, son sel ou un mélange de ceux-ci.

7. Bloc de savon selon l'une quelconque des revendications 1 à 6, dans lequel au moins un composant B est constitué par de l'acide laurique, de l'acide palmitique, de l'acide stéarique, leurs sels ou leurs mélanges.

8. Bloc de savon selon l'une quelconque des revendications 1 à 7, dans lequel au moins un composant C est constitué par de l'iséthionate de lauroyle sodique, de l'iséthionate de cocoyle sodique ou leur mélange.

9. Bloc de savon selon l'une quelconque des revendications 1 à 8, dans lequel ledit au moins un composant F est choisi dans le groupe constitué par les conservateurs, les parfums, les colorants, les tensioactifs, les polymères cationiques, les pigments, les agents surgraissants, les agents actifs antimicrobiens et biogènes, les agents hydratants, les stabilisateurs, les acides et les lessives.

10. Bloc de savon selon l'une quelconque des revendications 1 à 9, qui consiste en une composition cosmétique ou dermatologique.

11. Utilisation du bloc de savon selon l'une quelconque des revendications 1 à 10 en tant que combibar ou syndet.

12. Utilisation du bloc de savon selon l'une quelconque des revendications 1 à 10 pour le traitement ou le soin de la peau, des cheveux ou de la peau et des cheveux.

13. Procédé de fabrication d'un bloc de savon selon l'une quelconque des revendications 1 à 10, dans lequel les composants A, B, C, D, E et éventuellement F
